Europäisches Patentamt

European Patent Office

Office européen des brevets

(11) Veröffentlichungsnummer: **0 307 895**

**A2**

(12) # EUROPÄISCHE PATENTANMELDUNG

(21) Anmeldenummer: **88115038.7**

(22) Anmeldetag: **14.09.88**

(51) Int. Cl.4: **A61M 1/36**

(30) Priorität: **15.09.87 DE 3730981**

(43) Veröffentlichungstag der Anmeldung:
**22.03.89 Patentblatt 89/12**

(84) Benannte Vertragsstaaten:
**AT BE CH DE ES FR GB GR IT LI LU NL SE**

(71) Anmelder: **Lambrecht, Peter**
**Max-Rüttgers-Strasse 29**
**D-8021 Irschenhausen(DE)**

Anmelder: **Blanck, Oliver, Dipl.-Ing.**
**Max-Rüttgers-Strasse 29**
**D-8021 Irschenhausen(DE)**

(72) Erfinder: **Lambrecht, Peter**
**Max-Rüttgers-Strasse 29**
**D-8021 Irschenhausen(DE)**
Erfinder: **Blanck, Oliver, Dipl.-Ing.**
**Max-Rüttgers-Strasse 29**
**D-8021 Irschenhausen(DE)**

(74) Vertreter: **Patentanwälte Grünecker,**
**Kinkeldey, Stockmair & Partner**
**Maximilianstrasse 58**
**D-8000 München 22(DE)**

(54) **Mikrowellengerät.**

(57) Bei üblichen Mikrowellengeräten dieser Art sind durch manuell zu betätigende Haltevorrichtungen für den Beutel die Bedienung umständlich und eine innige Durchmischung des Beutelinhalts gefährdet, weil der Zirkulation des Beutelinhalts Strömungshindernisse entgegenstehen. Das neue Mikrowellengerät (1) soll unabhängig von der Beutelgröße bedienungsfreundlich sein und eine innige Mischung des Beutelinhalts gewährleisten.

Aus diesem Grund weist die Aufnahmevorrichtung (7) eine oben offene, flache Schale (8) mit einem umlaufenden Stützrand (10) auf, in der der Beutel (2) mit freibleibender Oberseite (6) allein durch den Stützrand (10) lagesicherbar ist. Mit der offenen Schale (8) ist eine einfache Bedienung gegeben. Durch die freibleibende Oberseite (6) stellt sich eine permanente wirkungsvolle Volumensverlagerung ein, die zu einer innigen Mischung des Beutelinhalts (5) führt.

Das Mikrowellengerät (1) eignet sich aufgrund des hohen Bedienungskomforts und der guten Qualität des erwärmten Beutelinhalts sowohl für wissenschaftliche als auch für ambulante Anwendungsfälle.

FIG.1

## Mikrowellengerät zum Erwärmen von Blut oder Blutbestandteilen in einem Beutel

Die Erfindung betrifft ein Mikrowellengerät der im Oberbegriff des Patentanspruchs 1 angegebenen Art.

Bei einem aus der DE-OS 35 02 095 bekannten Mikrowellengerät dieser Art weist die Aufnahmevorrichtung eine ebene Platte mit glatter Oberfläche auf, auf der der Beutel mit seiner Unterseite aufliegt und durch ein die Beuteloberseite überquerendes Spannband lagegesichert wird. Durch das Spannband wird der Beutel in zwei über eine Engstelle miteinander strömungsverbundene Blasen unterteilt, wobei die Engstelle eine ausreichende Durchmischung des Beutelinhaltes erschwert. Eine gleichmäßige Durchmischung des Beutelinhaltes ist aber für die Qualität des Beutelinhalts in erwärmtem Zustand unabdingbar. Das Spannband muß relativ fest gezogen sein, weil der Beutel wegen des permanenten Drehrichtungswechsels der Aufnahmevorrichtung sonst herabfallen würde. Neben dem unbefriedigenden Mischeffekt ist auch die umständliche Handhabung des Mikrowellengerätes nachteilig, weil zum Lagesichern des Beutels und zu seiner Entnahme stets mehrere Handgriffe notwendig sind.

Ferner ist es aus der DE-OS 35 308 bekannt, auf die Oberseite des auf einer ebenen Platte liegenden Beutels einen Plattenkörper aufzulegen und das Spannband von der Platte über den Plattenkörper zu spannen, so daß der Beutel unter Flachdrücken lagegesichert wird. Der dabei erzielbare Mischeffekt ist unbefriedigend, weil sich die Innenwände des flachgedrückten Beutels praktisch wie bei einem starren Behälter verhalten und den Bewegungen des Beutelinhalts nicht nachgeben. Die Handhabung ist außerordentlich kompliziert, weil beim Einlegen und Entnehmen des Beutels jeweils sowohl die Platte als auch das Spannband betätigt werden müssen.

Der Erfindung liegt die Aufgabe zugrunde, ein Mikrowellengerät der eingangs genannten Art zu schaffen, das bequem zu bedienen ist und aufgrund eines gleichbleibend guten Mischeffekts zu einer hohen Qualität des erwärmten Beutelinhalts führt.

Die gestellte Aufgabe wird erfindungsgemäß mit den im kennzeichnenden Teil des Patentanspruchs 1 angegebenen Merkmalen gelöst.

In die oben offene Schale wird der Beutel nur mit einem Handgriff eingelegt. Dank des umlaufenden Stützrandes gleitet er beim Erwärmen nicht von der Aufnahmevorrichtung herab. Zur Entnahme ist ebenfalls nur ein Handgriff notwendig. Da nichts auf die Oberseite des Beutels drückt, ist die Oberseite des Beutels frei beweglich, so daß beim Drehen der Schale eine wirksame Durchmischung des Beutelinhaltes erfolgt, aus der dank der gleichmäßigen Erwärmung ohne lokale Überwärmungen eine hohe Qualität des erwärmten Beutelinhalts resultiert. Eventuell eingeschlossene Gasblasen können ungehindert aufsteigen und unterstützen die Durchmischung. Ein Teil des Beutelinhaltes fließt bei der Drehbewegung der Aufnahmevorrichtung stets zum tiefer liegenden Bereich der Schale und vermischt sich innig mit dem anderen Teil des Beutelinhalts. Es entsteht eine permanente Zirkulation. Die freie Oberseite des Beutels folgt den Schwall- oder Fließbewegungen des Beutelinhalts und stellt kein Strömungshindernis bei der Mischungszirkulation dar. Als Folge dieser vorteilhaften Wirkungen der Schale auf den Beutel reicht eine relativ langsame Drehbewegung mit gleichbleibender Geschwindigkeit und gleichbleibender Drehrichtung für eine innige Mischung aus, was zu einer Vereinfachung des Drehantriebs und der Steuervorrichtungen für den Drehantrieb führt. Neben Blut und Blutbestandteilen lassen sich auch Infusionen, Transfusionen, Sperma, Transplantate, Frischzellen-Präparate od. dgl. jeweils individuell und schonend erwärmen.

Es ist zwar aus einem Prospekt eHAEMOTHERMe der Firma Robert Bosch GmbH ein Mikrowellengerät bekannt, bei dem die Aufnahmevorrichtung eine offene Schale, jedoch mit senkrechter Auflagefläche aufweist, die zur formschlüssigen Lagesicherung einer Flasche konzipiert ist und zusätzlich auch das Aufnehmen eines Beutels gestattet. Jedoch ist die Schale der Form der Flasche angepaßt und wird zum Aufnehmen der Flasche oder des Beutels ein Spannband mit einem Druckkissen über den jeweiligen Behälter gespannt, weil sonst wegen der senkrechten Lage der Schale die Flasche oder der Beutel herausfallen würde. Da die Form der Schale nicht dem Beutel entspricht, werden die Beutelunterseite und auch die Beuteloberseite durch das Spannband und das Druckkissen unzweckmäßig verformt. Die Handhabung dieses Mikrowellengerätes ist beim Einlegen und Entnehmen des Behälters umständlich. Der Mischeffekt leidet bei einem Beutel unter den Verformungen. Die vorgesehene Schale mit der Flaschenform benötigt wegen der vertikalen Anordnung eine Hilfsvorrichtung zum Lagesichern des jeweiligen Behälters.

Einem zu weiten Verrutschen des Beutels auf der Auflagefläche wird bei der Ausführungsform von Anspruch 2 von vornherein entgegengewirkt.

Um zu verhindern, daß bei der Drehbewegung der Aufnahmevorrichtung ein Teil des Beutels über den Stützrand überhängt, sind die Maßnahmen von Anspruch 3 wichtig. Der Stützrand kann auch flacher gestaltet sein, weil er eine wirksame Barriere gegen das Verrutschen des Beutels ist.

Die Ausführungsform gemäß Anspruch 4 zeichnet sich durch einen einfachen apparativen und steuerungstechnischen Aufwand auf. Ein einfacher Drehantrieb läßt sich deshalb verwenden, weil durch die Beweglichkeit der freibleibenden Oberseite des Beutels auch bei konstanter Drehgeschwindigkeit und gleichbleibender Drehrichtung eine innige Mischung des Beutelinhaltes erzielt wird. Der Motor läuft zufällig in einer Richtung an und behält diese Drehrichtung bei.

Eine besonders wichtige Maßnahme geht aus Anspruch 5 hervor. Der Rotor sorgt für eine Inhomogenität der Mikrowellenbeaufschlagung, die sicherstellt, daß der Beutelinhalt nicht lokal in Wellenknoten oder Wellenbergen zu wenig oder zu stark erwärmt wird.

Günstig ist dabei auch die Maßnahme von Anspruch 6, weil auch aufgrund der Wechselwirkung der Drehzahlen und/oder der Drehrichtungen die vorerwähnte Gefahr beseitigt wird.

Auf baulich einfache Weise läßt sich das vorerwähnte Ziel auch bei der Ausführungsform gemäß Anspruch 7 realisieren. Das Energiefeld muß zwangsweise den Flügelrotor passieren, der eine Ablenkung und Zerstreuung bewirkt, deren Intensität durch die Drehbewegung des Flügelrotors noch verstärkt wird.

In der Praxis hat sich eine Ausführungsform bewährt, wie sie aus Anspruch 8 hervorgeht. Die langsame Drehgeschwindigkeit der Aufnahmevorrichtung gewährleistet die schwerkraftabhängige innige Mischung des Beutelinhalts und vermeidet ein Aufschäumen bzw. zu rasches Schwappen des Beutelinhalts.

Wichtig ist auch die Maßnahme von Anspruch 9, weil der gerundete Übergang der Mischströmung im Beutel zugute kommt. In einfacheren Geräten kann der Übergang auch eckig sein.

Eine weitere, zweckmäßige Maßnahme ist aus Anspruch 10 zu entnehmen. Mit der reibungsfreudigen Beschichtung und/oder der Strukturierung wird die Lagesicherung des Beutels in der Schale verbessert, so daß der Neigungswinkel der Auflagefläche vergrößert werden kann.

Eine weitere wichtige Ausführungsform, bei der in der Auflagefläche ein Temperaturfühler vorgesehen ist, geht aus Anspruch 11 hervor. Der konvex gekrümmte Fühlkopf hat eine relativ große Abtastfläche für die Unterseite des Beutels, um die Temperatur des Beutelinhalts exakt abzugreifen. Dazu kommt ein wünschenswerter Einfluß des vortretenden, konvex gekrümmten Fühlkopfes auf die Mischung des Beutelinhalts bei der Drehung der Aufnahmevorrichtung, weil der Fühlkopf eine sanfte Krümmung der Unterseite des Beutels erzeugt, über die hinweg der Beutelinhalt bei jeder Umdrehung kurzzeitig beschleunigt und umgelenkt wird.

Wichtig ist auch das Merkmal von Anspruch 12, weil bei dieser Position des Fühlkopfes die Begünstigung der Mischung am besten und der ermittelte Temperaturwert am exaktesten ist. Wäre der Fühlkopf exzentrisch zur Drehachse der Schale angeordnet, so bestünde die Gefahr, daß er bei jeder Umdrehung Temperaturschwankungen feststellen würde, die die Auswertung der tatsächlichen Temperatur erschweren könnten.

Damit der Fühlkopf, der z.B. aus Kupfer ist, nicht anläuft, ist die Maßnahme von Anspruch 13 wichtig.

Schließlich geht eine weitere, besonders wichtige Ausführungsform aus Anspruch 14 hervor. Die Verbrauchsqualität des erwärmten Beutelinhalts ist das wichtigste Kriterium. Der Beutelinhalt ist nämlich außerordentlich empfindlich gegen Überhitzung, d.h. sowohl gegen lokale Überhitzung als auch gegen eine zu rasche Erwärmung. Durch die über eine Steuerschaltung hergestellte Kopplung zwischen dem Temperaturfühler und dem Mikrowellenerzeuger wird von vornherein nur ein bestimmter Temperaturanstieg über der Zeit zugelassen, um eine zu rasche Erwärmung auszuschließen. Dies ist besonders im Hinblick darauf wichtig, daß mit dem Mikrowellengerät auch Beutel unterschiedlicher Größen oder Fassungsvermögen behandelt werden, wobei die Leistungfähigkeit des Gerätes auf die größten zu behandelnden Beutel abgestimmt wird, und bei kleineren Beuteln durch das zeitweise Aussetzen der Mikrowellenbeaufschlagung eine bestimmte Erwärmungsgeschwindigkeit eingehalten wird. Die Abschaltzeitdauer kann individuell hochgerechnet werden.

Beim bereits erwähnten Mikrowellengerät gemäß DE-OS 35 43 308 sind die Abschirmflächen auf der Auflagefläche der Aufnahmevorrichtung und an der Unterseite der auf den Beutel aufgepreßten Platte vorgesehen, so daß der Beutel im abgeschirmten Bereich flachgequetscht wird. In der Praxis ist dieses bekannte Mikrowellengerät soweit modifiziert, daß an beiden Längsenden der Aufnahmefläche federnd gegen die Auflagefläche gepreßte Klappen mit Griffbügeln angelenkt sind, die Abschirmflächen aufweisen. Abgesehen davon, daß die Klappen den Beutel unzweckmäßig deformieren, ist die Handhabung beim Einlegen und Entnehmen eines Beutels außerordentlich umständlich, weil zunächst die Klappen mit beiden Händen in die Öffnungsstellung gebracht und in dieser gehalten oder fixiert werden müssen, ehe der Beutel eingelegt werden kann. Nach dem Einlegen des Beutels sind die beiden Klappen unter Festhalten des Beutels wieder umzulegen. Dafür ist ferner eine bestimmte Drehposition der Auflagefläche nötig. Bei der Ausführungsform gemäß Anspruch 15 ist trotz der Abschirmflächen eine einfache Handhabung gegeben, weil die Platte mit der einen Hand und unabhängig von der Drehposition der Auflagefläche in die Beuteleinlegestellung gebracht wird, während der Beutel mit der anderen Hand auf die Auflagefläche gelegt wird. Die Platte wird nicht benötigt, um den Beutel auf der Auflagefläche zu positionieren, so daß dessen Oberseite nicht in einem die Zirkulation beeinflussenden Maß verformt wird, wenn sich die Platte in der Abschirmstellung befindet. In der Abschirmstellung der Platte können die Mikrowellen im Anschlußbereich des Beutels und in den dort zwangsweise vorliegenden dünnen Stellen oder Winkeln den Beutelinhalt nicht mehr lokal überhitzen. Dank der Zirkulation und der innigen Mischung des Beutelinhalts aufgrund der freibeweglichen Oberseite des Beutels wird der Beutelinhalt auch in diesem Bereich ausreichend erwärmt, ohne daß eine direkte Beaufschlagung durch die Mikrowellen erforderlich wäre.

Eine besonders einfache Bedienung ist bei der Ausführungsform gemäß Anspruch 16 gegeben, weil sich die Platte leicht zwischen den beiden Stellungen hin-und herklappen läßt.

Günstig ist ferner die Ausführungsform gemäß Anspruch 17, weil an den Anschlägen die Platte in der jeweiligen Position abgefangen wird. In der Abschirmstellung wird vermieden, daß die Platte einen nennenswerten Verformungsdruck auf die Oberseite des Beutels ausübt, weil sie an den Anschlägen abgefangen ist. Eine Verlagerung aufgrund der Trägheitskräfte bei der Drehbewegung der Aufnahmevorrichtung ist nicht zu befürchten.

Im Hinblick auf die Bedienungsfreundlichkeit ist ferner die Ausführungsform gemäß Anspruch 18 zweckmäßig. Wird bei in der Beuteleinlegestellung befindlicher Platte nicht bereits durch den Einlegevorgang mit der gegen den kleineren Plattenteil stoßenden Beutelseite die Klappe in die Abschirmstellung geklappt, dann wird bei der Drehbewegung aufgrund der Schräglage der Aufnahmefläche durch Schwerkrafteinwirkung die Klappe selbsttätig rasch in die Abschirmstellung fallen.

Baulich einfach ist die Ausführungsform gemäß Anspruch 19.

Damit reflektierte Mikrowellen den Anschlußbereich des Beutels nicht ungewollt beaufschlagen, ist ferner die Ausführungsform gemäß Anspruch 20 zweckmäßig. Die Abschirmplatte an der Unterseite der Schale läßt sich dort einfach befestigen, sie drückt sich nicht in die Beutelunterseite ein.

Da aufgrund unterschiedlicher Beutelinhalte, unterschiedlicher Vorschriften oder unterschiedlicher Anwendungsfälle die Beutelgröße stark variieren kann, und da andererseits eine zu starke Wanderbewegung des Beutels auf der Auflagefläche im Hinblick auf die einwandfreie Temperaturermittlung unerwünscht ist, z.B. damit der Anschlußbereich unter den Abschirmflächen bleibt, ist die Ausführungsform gemäß Anspruch 21 von besonderer Bedeutung. Denn es wird hier eine individuelle Anpassung des Mikrowellengerätes an die jeweilige Beutelgröße gestattet. In Abhängigkeit von der Länge des zu bearbeitenden Beutels wird die freie Länge der Auflagefläche verkürzt oder vergrößert, so daß der Beutel genau auf die Auflagefläche paßt.

Besonders zweckmäßig ist hierfür die Ausführungsform gemäß Anspruch 22, bei der die Auflagefläche auf die Größe des größten, vorkommenden Beutels abgestimmt ist, und sich bei der Bearbeitung kleinerer Beutel entsprechend verkleinern läßt, indem das Querteilelement in Längsrichtung verstellt wird.

Baulich besonders einfach, praxisgerecht und bedienungsfreundlich ist ferner die Ausführungsform gemäß Anspruch 23. Der Klemmbügel bildet sozusagen einen verstellbaren Stützrand für kleinere Beutelgrößen, der sich auf den seitlichen Stützrändern abstützt und daran verschoben werden kann. Die Einstellung auf die jeweilige Beutelgröße ist praktisch nur ein Handgriff.

Eine preiswerte und funktionssichere Lösung geht aus Anspruch 24 hervor. Die W-Form des Klemmbügels verhindert, daß ein kleinerer Beutel unter dem Bügel durchrutschen könnte. Die Klammerenden sichern eine ausreichende Halterung des Klemmbügels in seiner jeweiligen Lage. Bei großen Beuteln, die die volle Länge der Auflagefläche ausnutzen, läßt sich der Klemmbügel einfach wegnehmen.

Zweckmäßig ist dazu die Ausführungsform gemäß Anspruch 25, weil mittels der Abdrücklaschen das Entfernen des Klemmbügels leicht durchführbar ist, und weil sich auch das Verstellen des Klemmbügels mittels der Abdrücklaschen, z.B. durch geringfügiges Lockern des Klammersitzes des Klemmbügels, leicht verstellen läßt. Es ist allerdings auch denkbar, den Klemmbügel mit einer Verrastung oder mit Halteelementen an der Schale festzulegen.

Günstig ist ferner die Ausführungsform gemäß Anspruch 26, weil mit den Randflanschen für den tiefgefrorenen Beutel unter Umständen gefährliche Kanten an den Stützrändern vermieden sind, und weil der Klemmbügel eine großflächige und sichere Halterung auch an den Randflanschen finden kann.

Anhand der Zeichnung werden Ausführungsformen des Erfindungsgegenstandes erläutert. Es zeigen:

Fig. 1 einen Vertikalschnitt durch ein Mikrowellengerät,

Fig. 2 einen Detailschnitt in der Ebene II-II von Fig. 1,

Fig. 3 einen Detailschnitt in der Ebene III-III von Fig. 1,

Fig. 4 einen Detailschnitt, ähnlich dem von Fig. 1, einer anderen Ausführungsform, und

Fig. 5 einen Teilschnitt durch eine weitere modifizierte Ausführungsform.

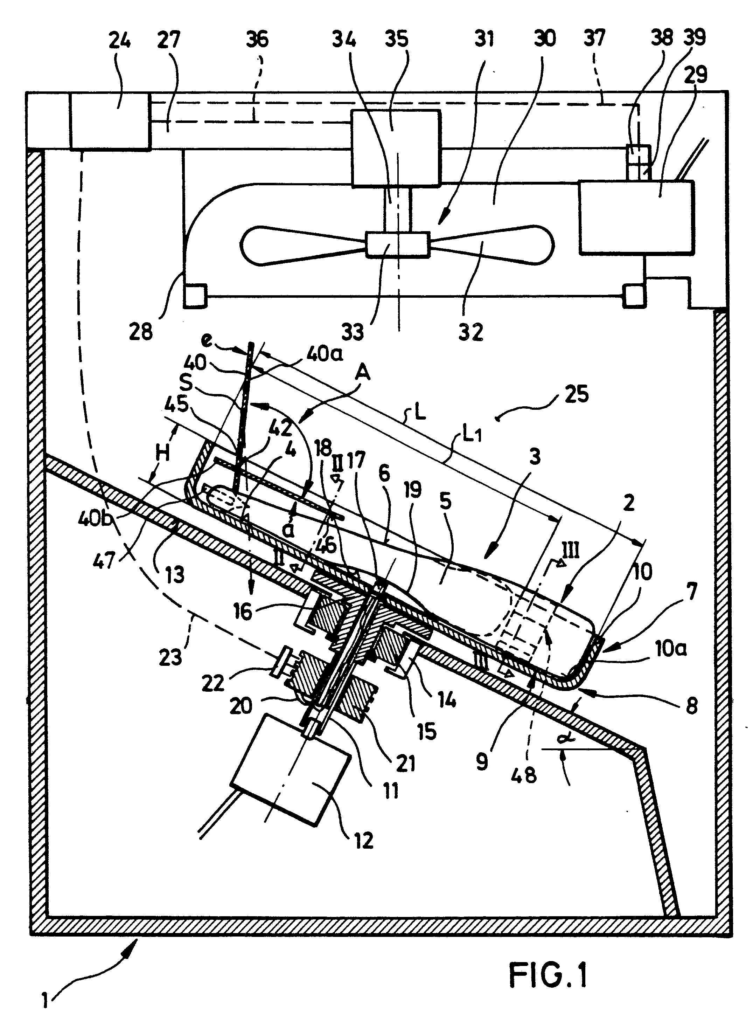

Ein Mikrowellengerät 1, das gemäß Fig. 1 zum Erwärmen von in einem Beutel 2 enthaltenem Blut oder Blutbestandteilen, wie Vollblut, aufgeschwemmten Erythrozytenkonzentraten und Fresh Frozen Plasma dient, kann als Standgerät, Wandgerät oder fahrbares Gerät ausgebildet sein.

Der Beutel 2 besteht aus einem weich-elastischen Kunststoff. Er hat viereckige Gestalt mit einer Unterseite 4 und einer Oberseite 6. Der Beutelinhalt 5 ist im Beutel 2 hermetisch dicht eingeschlossen. Nahe zumindest eines Längsrandes des Beutels 2 weist dieser einen Anschlußbereich 47 auf. In diesem sind eingeformte und verschweißte Rohr- oder Schlauchstutzen zum Befüllen bzw. Entleeren und gegebenenfalls ein Aufhänger vorgesehen.

Eine Aufnahmevorrichtung 7 weist eine oben offene, flache Schale 8 mit einer ebenen oder leicht gewölbten Auflagefläche 9 sowie einen umlaufenden Stützrand 10 auf. Die Form der Auflagefläche 9 und der Verlauf des Stützrandes 10 entsprechen dem Umfangsverlauf des Beutels 2.

Die Schale 8 ist an einem Ende eines Wellenrohres 11 befestigt, dessen anderes Ende mit einem Drehantrieb 12 gekuppelt ist, der zweckmäßigerweise ein Elektromotor mit konstanter Drehgeschwindigkeit und gleichbleibender Drehrichtung ist. Die Hohlwelle 11 ist in einem unter einem Neigungswinkel gegenüber der Horizontalen gezeigten Bodenteil 13 in einer Halterung 14 mit einem Drehlager 15 gelagert, das eine Nabe 16 aufnimmt, an der die Schale 8, gegebenenfalls austauschbar, befestigt ist. Nahe der Auflagefläche 9 ist in der Hohlwelle 11 ein Temperaturfühler 17 untergebracht, der mit einem pilzförmigen Fühlkopf 18 ausgestattet ist, der in der Mitte der Auflagefläche 9 und konvex gekrümmt über diese überstehend angeordnet ist. Der Fühlkopf 18 (z.B. aus Kupfer) ist zweckmäßigerweise mit einer das Anlaufen verhindernden Beschichtung 19, z.B. aus einem Edel- oder einem Buntmetall, versehen.

Auf der Hohlwelle 11 sitzt ein Schleifring 21, an den Anschlußleitungen 20 vom Temperaturfühler 17 herangeführt sind. Schleifkontakte 22 berühren den Schleifring 21 und stehen über eine Signalleitung 23 mit einer Schaltsteuereinrichtung 24 in Verbindung, die im oberen Teil des Mikrowellengerätes 1 untergebracht sein kann. Im Mikrowellengerät 1 ist oberhalb des Bodens 13 ein Erwärmungsraum 25 vorgesehen, der nach außen durch eine Tür 26 verschlossen ist. Nach Öffnen der Tür 26 kann der Beutel in Richtung eines Pfeiles 3 in die Schale 8 eingelegt oder in der entgegengesetzten Richtung aus dieser entnommen werden.

In der mit 27 bezeichneten Deckwand des Mikrowellengerätes 1 ist ein Einbauteil 28 angebracht, das einen Mikrowellenerzeuger 29, z.B. ein Magnetron, enthält. Von diesem erstreckt sich durch den Einbauteil 28 ein Einspeisungskanal 30 bis zur nach unten freien Öffnung des Einbauteils 28. Im Einspeisungskanal 30 ist ein Rotor 31 angeordnet, der aus an einer Nabe 33 befestigten Flügeln 32 besteht und an einer Welle 34 angebracht ist, die mit einer Drehantriebsvorrichtung 35 in Verbindung steht. Die Drehantriebsvorrichtung 35 kann über eine Steuerleitung 36 an die Schaltsteuereinheit 24 angeschlossen sein. Eine weitere Signalleitung 37 führt von der Schaltsteuereinheit 24 über eine Schalteinrichtung 38 mit einem Zeitglied 39 zum Mikrowellenerzeuger 29. In der Schaltsteuereinrichtung 24 ist zweckmäßigerweise ein Mikrocomputer zur Steuerung enthalten.

Es lassen sich Beutel mit verschiedenen Fassungsvermögen, z.B. zwischen 200 und 750 ml, behandeln. Die Dauer der Erwärmung ist je nach Fassungsvermögen des Beutels und Leistung des Magnetrons verschieden, z.B. zwischen 60 und 150 Sekunden. Die Temperatur des Beutelinhalts 5 wird mittels einer nicht dargestellten Anzeigevorrichtung, z.B. einem zweistelligen Display, fortlaufend angezeigt.

Der Anschlußbereich 47 des Beutels 2 wird zweckmäßigerweise gegen die Mikrowellen abgeschirmt, um lokale Überhitzungen in den dort vorliegenden Winkeln oder dünneren Stellen des Beutels zu vermeiden. Zu diesem Zweck sind Abschirmflächen A nahe einem Stützrand 10 der Schale 8 vorgesehen. Im vorliegenden Ausführungsbeispiel ist eine der Oberseite 6 des Beutels 2 zugeordnete Abschirmfläche durch eine Platte 40 aus einem für Mikrowellen undurchlässigen Material, z.B. Metall oder Buntmetall gebildet, die an der Schale 8 zwischen einer Beuteleinlegestellung e und einer Abschirmstellung a in Richtung eines Doppelpfeiles um eine parallel zur Auflagefläche 9 verlaufende Scharnierachse 42 schwenkbar. An der Unterseite der Schale 8 ist eine weitere Abschirmplatte 41 fest angebracht. Diese kann gegebenenfalls in die Schale 8 eingeformt oder auch bündig in der Auflagefläche 9 vorgesehen sein.

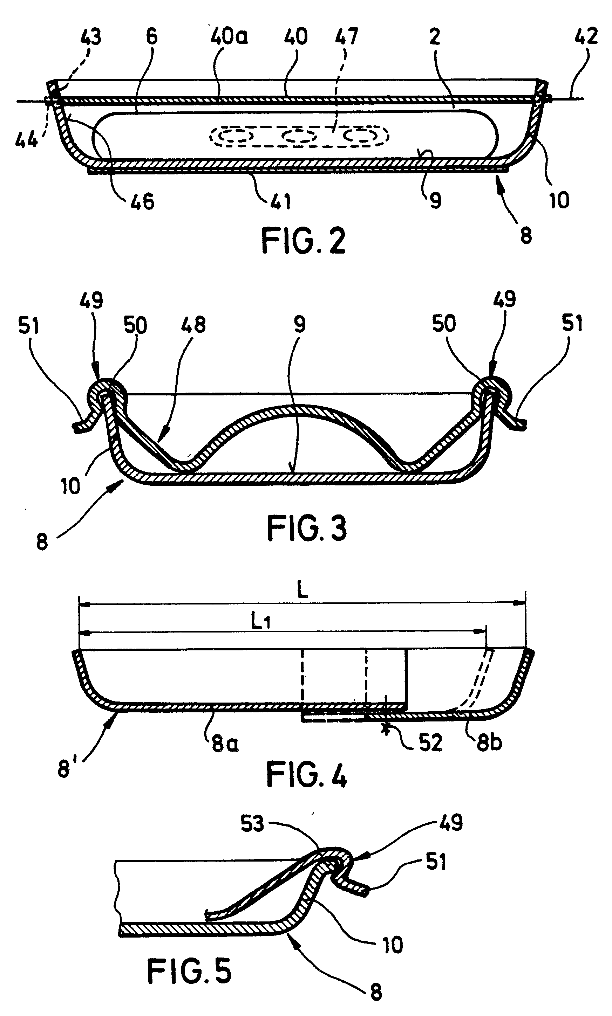

Die Klappe 40 (Fig. 2) ist um ein Scharnier klappbar, das aus an den Seitenrändern der Klappe angeordneten Zapfen 44 und Ausnehmungen 43 in sich gegenüberliegenden Stützrändern 10 der Schale 8 besteht. Die Scharnierachse 42 liegt zweckmäßigerweise geringfügig unterhalb der freien Randkante 50 (Fig. 3) der Stützränder 10.

Die Abschirmplatte 40 besitzt einen größeren Plattenteil 40a und einen kleineren Plattenteil 40b. Durch den Verlauf der Stützränder 10 sowie durch deren sich nach oben öffnende Schräge werden Anschläge 45 und 46 gebildet, an denen die Platte 40 sowohl in der Beuteleinlegestellung e als auch in der Abschirmstellung a abgefangen ist. Die Anschläge 46 sind zweckmäßigerweise so angeordnet, daß die Platte 40 in der Abschirmstellung überhaupt nicht oder nur geringfügig auf den Beutel 2 drückt. Hingegen sind die Anschläge 45 so angeordnet, daß der kleinere Plattenteil 40b in der Beuteleinlegestellung e vom vorderen Rand des Beutels 2 beim Einlegen getroffen wird, so daß die Platte 40 selbsttätig in die Abschirmstellung a klappt. Zusätzlich sind die Anschläge 45 so angeordnet, daß der Massenschwerpunkt S der Platte 40 bzw. des größeren Plattenteils 40a in der Beuteleinlegestellung e in einer Vertikalprojektion an der Seite der Scharnierachse 42 liegt, zu der der größere Plattenteil 40a bei der Bewegung der Platte 40 in die Abschirmstellung a bewegt werden muß. Damit wird erreicht, daß aufgrund der Schräglage (Winkel α) der Auflagefläche 9 die Platte 40 selbsttätig in die Abschirmstellung a klappt, sobald die Schale 8 entsprechend weit verdreht wird.

Die freie Länge L der Auflagefläche 9 zwischen den Stützrändern 10 ist auf die größte zu erwartende Beutelgröße abgestimmt. Damit kleinere Beutel 2 nicht unkontrolliert auf der Auflagefläche hin- und herrutschen, ist die freie Länge entsprechend verkürzbar (L1). Zu diesem Zweck ist ein Querteilelement 48 vorgesehen, das sich in Längsrichtung der Schale 8 verstellen läßt und dann den Stützrand für den jeweils kleineren Beutel bildet. Fig. 3 verdeutlicht, daß das Querteilelement 48 als im Grundzug W-förmiger Klemmbügel ausgebildet ist, der sich quer durch die Schale 8 erstreckt und mit Klammerenden 49, die die Kante des Stützrandes 10 von oben übergreifen, an sich gegenüberliegenden Stützrändern festgeklemmt ist, so daß er für eine einwandfreie Abstützung des Beutels sorgt. An zumindest einem Klammerende ist eine nach außen ragende Abdrücklasche 51 angeformt, die das Verschieben des Klemmbügels vereinfacht und mit der auch der Klammergriff des Klemmbügels leicht lösbar ist, entweder zum Verstellen oder zum Abnehmen des Klemmbügels. Der Klemmbügel ist zweckmäßigerweise ein Kunststofformteil, z.B. ein entsprechend verformter Thermoplast- oder Duroplast-Streifen.

Fig. 4 zeigt eine abgewandelte Ausführungsform einer Schale 8', deren maximale, freie Länge L dadurch auf einen neuen Wert L1 verstellt werden kann, daß die Schale aus zwei teleskopartig relativ zueinander verschiebbaren Schalenteilen 8a und 8b besteht, die in der jeweils eingestellten Länge durch Elemente 52 festgelegt werden können.

Das Mikrowellengerät 1 wird wie folgt betrieben:

Zunächst ist zu überprüfen, daß am Beutel keine Metallteile, Klammern od.dgl., vorhanden sind. Dann wird die Tür 26 geöffnet, wobei eine nicht dargestellte Innenraumbeleuchtung eingeschaltet wird. Der Beutel 2 wird, nachdem die Klappe 40 angehoben wurde, in Richtung des Pfeiles 3 in die Schale 8 eingelegt, derart, daß der Anschlußbereich 47 unter der Klappe 40 liegt und die Unterseite 4 des Beutels 2 den Fühlkopf 18 abdeckt. Eine zusätzliche Lagesicherung des Beutels 2 ist nicht erforderlich. Nach dem Schließen der Tür 26 wird automatisch der Drehantrieb 12 eingeschaltet, wobei die Innenraumbeleuchtung angeschaltet bleibt. Es wird dann z.B. über den raschen Temperaturabfall, festgestellt, ob tatsächlich ein Beutel 2 eingelegt ist. Ist dies nicht der Fall, so wird der Drehantrieb 12 nicht aktiviert. Ist die Klappe 40 nicht bereits durch den am Klappenteil 40b anstoßenden Beutel 2 eingelegt, dann fällt sie bei der Umdrehung durch Schwerkrafteinwirkung herunter.

Sofort nach dem Einlegen des Beutels mißt der Temperaturfühler 17 die Anfangstemperatur des Beutelinhalts 5. Diese Temperatur wird angezeigt.

Nach dem Schließen der Tür wird ein nicht dargestellter Startknopf gedrückt, wodurch die Drehantriebsvorrichtung 35 für den Rotor 31 in Gang gesetzt wird. Mit einer kurzen Verzögerung, z.B. 3 Sekunden, wird der Mikrowellenerzeuger 29 aktiviert. Das Energiefeld wird durch den Rotor 31 zu einer Rotation veranlaßt.

Bei der relativ langsamen Drehung der Schale 8 (mehrere Umdrehungen pro Minute) wird der Beutelinhalt 5 sanft und gleichmäßig wirksam durchmischt. Es fließt vom höher liegenden Bereich der Auflagefläche fortwährend ein Teil des Beutelinhalts 5 zum tiefer liegenden Bereich, wobei sich die Oberseite 6 des Beutels 2 der Zirkulationsbewegung anpaßt und der Beutelinhalt 5 über die Erhöhung im Bereich des Fühlkopfes 18 beschleunigt wird. Dabei legt sich der Beutel jeweils im tiefer liegenden Bereich der Schale 8 an den Stützrand 10 an, während er im höheren Bereich vom Stützrand abweicht. Die Höhe H des Stützrands 10 ist so bemessen, daß der Beutel 2 nicht über den Stützrand hinweghängen kann. Der Übergang 10a von der Auflagefläche 9 zum Stützrand 10 ist konkav gerundet, um auch hier einwandfreie Zirkulationsbewegungen zuzulassen. Die Rotation des Energiefeldes erfolgt mit ungefähr 1 Umdrehung pro Sekunde und gegebenenfalls mit einer der Drehrichtung der Schale 8 entgegengesetzten Drehrichtung. Durch die innige Durchmischung des Beutelinhalts 5 erfolgt eine gleichmäßige Erwärmung ohne die Gefahr von lokalen Heiß-oder Kaltstellen.

Die Erwärmung wird fortgesetzt, bis eine Endtemperatur von beispielsweise 36°C im Beutelinhalt 5 erreicht ist.

Die Geschwindigkeit der Erwärmung des Beutelinhalts 5 wird begrenzt, um die Qualität des erwärmten Beutelinhalts nicht zu verschlechtern.

Erwärmt sich der Beutelinhalt schneller als beispielsweise 1°C in zwei Sekunden, so wird über die Schaltsteuereinheit 24 und die Schalteinrichtung 38 sowie das Zeitglied 39 der Mikrowellenerzeuger 29 abgeschaltet. Das Zeitglied 39 hält eine Zeitperiode von beispielsweise fünf Sekunden ein, ehe der Mikrowellenerzeuger 29 wieder eingeschaltet wird. Am Ende der Erwärmvorganges leuchtet eine nicht dargestellte Kontrolleuchte auf und ertönt ein akustisches Signal.

Danach wird nach Öffnen der Tür 26 der Beutel 2 mit dem erwärmten Beutelinhalt entnommen und verarbeitet.

Das Mikrowellengerät 1 hat eine automatische Warmhaltefunktion. Nach Erreichen der gewünschten Endtemperatur, z.B. 36°C, wird der Mikrowellenerzeuger 29 abgeschaltet. Der Drehantrieb 12 bleibt weiterhin aktiviert. Sinkt danach die Temperatur des Beutelinhalts 5 beispielsweise wieder unter 33°C, so wird über den Temperaturfühler 17 und die Schaltsteuereinrichtung 24 automatisch der Mikrowellenerzeuger 29 wieder eingeschaltet.

Wie üblich wird der Mikrowellenerzeuger 29 beim Öffnen der Tür während des Erwärmvorganges automatisch abgeschaltet. Bei einem Defekt des Mikrowellengerätes oder bei einer Fehlbedienung (Start ohne eingelegten Beutel) oder wenn der Beutelinhalt über 38°C erwärmt worden ist, dann leuchtet eine Kontrolleuchte auf und ertönt ein akustisches Signal. Die Kontrolleuchte und das Warnsignal erlöschen nach Öffnen der Tür. Das Mikrowellengerät 1 ist dann wieder betriebsbereit. Handelt es sich um einen internen Fehler im Mikrowellengerät 1, so leuchtet die Kontrolleuchte weiter auf.

Da der Beutel durch keine Halterungen verformt wird, tritt eine gute Durchmischung des Beutelinhalts infolge permanenter großer Volumenverlagerungen während der Drehung der Schale 8 ein. Eine gleichmäßige Erwärmung wird durch die Überlagerung der voneinander unabhängigen Drehbewegungen des Energiefeldes einerseits und der Schale andererseits gewährleistet. Da die Schale 8 nur in einer Drehrichtung und mit gleichbleibender Drehgeschwindigkeit angetrieben wird, reicht ein einfacher und preiswerter Antrieb aus, für den keine Richtungsumschalter oder Geschwindigkeitssteuerungen erforderlich sind. Zu der guten Qualität des erwärmten Beutelinhalts kommt der Vorteil der außerordentlich einfachen Bedienung, weil der jeweils zu behandelnde Beutel nach Anheben der Platte 40 nur in die offene Schale 8 eingelegt zu werden braucht und genauso einfach daraus wieder entnommen werden kann. Die Schale 8 besteht zweckmäßigerweise aus Porzellan. Denkbar sind auch Kunststoffschalen oder gitterartige Schalen aus gegen Mikrowellen unempfindlichen Materialien. Es könnte auch eine ebene Platte mit entlang ihren Rändern hochstehenden Vorsprüngen verwendet werden. Die Auflagefläche 9 könnte auch rutschfest und/oder mit einer Struktur, gegebenenfalls konzentrischen Rippen oder Noppen, versehen sein. Der Rotor könnte auch mit einem Gebläse angetrieben werden, das gegebenenfalls gleichzeitig für ein gleichmäßiges Klima im Erwärmungsraum sorgt.

## Ansprüche

1. Mikrowellengerät (1) zum Erwärmen von in einem Beutel (2) bevorratetem Blut, Blutbestandteilen oder einer anderen, dem menschlichen Körper zuzuführenden Flüssigkeit, mit einem von einem Mikrowellenerzeuger (29) beschickbaren Erwärmungsraum (25), in dem eine eine Auflagefläche (9) für die Unterseite des Beutels (2) aufweisende Aufnahmevorrichtung (7) für den Beutel (2) drehbar gelagert und mit einem Drehantrieb (12) verbunden ist, wobei die Auflagefläche (9) gegenüber der Horizontalen geneigt ist, **dadurch gekennzeichnet, daß** die Aufnahmevorrichtung (7) eine oben offene, flache Schale (8, 8') mit einem umlaufenden, von der Auflagefläche (9) hochstehenden Stützrand (10) ist, in die der Beutel (2) frei einleg- und mit freibleibender Oberseite (6) allein durch den Stützrand (10) lagesicherbar ist.

2. Mikrowellengerät nach Anspruch 1, **dadurch gekennzeichnet, daß** die Neigung (α) der Auflagefläche (9) relativ zur Horizontalen annähernd dem Reibungswinkel zwischen der Beutelunterseite (4) und der Auflagefläche (9) entspricht.

3. Mikrowellengerät nach den Ansprüchen 1 und 2, **dadurch gekennzeichnet, daß** die Höhe (H) des Stützrandes (10) annähernd der Dicke des Beutels (2) entspricht.

4. Mikrowellengerät nach den Ansprüchen 1 bis 3, **gekennzeichnet durch** einen Drehantrieb (12) mit während eines Erwärmungsvorgangs gleichbleibender Drehrichtung und konstanter Drehgeschwindigkeit.

5. Mikrowellengerät nach einem der Ansprüche 1 bis 4, **dadurch gekennzeichnet, daß** zwischen dem Mikrowellenerzeuger (29) und der Aufnahmevorrichtung (7) ein mit einer Drehantriebsvorrichtung (35) verbundener oder durch ein Gebläse angetriebener Rotor (31) zum Drehen des Energiefeldes vorgesehen ist.

6. Mikrowellengerät nach Anspruch 5, **dadurch gekennzeichnet, daß** die Drehzahlen und/oder Drehrichtungen der Aufnahmevorrichtung (7) und des Rotors (31) verschieden sind.

7. Mikrowellengerät nach den Ansprüchen 5 und 6, **dadurch gekennzeichnet, daß** der Rotor (31) in einem Einspeiskanal (30) der Mikrowellen zum Erwärmungsraum (25) angeordnet ist und einen auf einer Welle (34) angebrachten Flügelrotor (32) aufweist.

8. Mikrowellengerät nach einem der Ansprüche 1 bis 7, **dadurch gekennzeichnet,** daß der Rotor (31) zu in etwa einer Umdrehung pro Sekunde und die Aufnahmevorrichtung (7) zu mehreren Umdrehungen pro Minute antreibbar sind.

9. Mikrowellengerät nach Anspruch 1, **dadurch gekennzeichnet,** daß der Übergang (10a) von der Auflagefläche (9) zum Stützrand (10) konkav gerundet ist.

10. Mikrowellengerät nach den Ansprüchen 1 bis 9, **dadurch gekennzeichnet,** daß die Auflagefläche (9) eine reibungsfreudige Beschichtung trägt und/oder mit einer Strukturierung, z.B. Rippen oder Noppen, versehen ist.

11. Mikrowellengerät nach einem der Ansprüche 1 bis 10, wobei in der Auflagefläche (9) ein Temperaturfühler (17) vorgesehen ist, **dadurch gekennzeichnet,** daß der Temperaturfühler (17) wenigstens einen über die Auflagefläche (9) vortretenden, konvex gekrümmten Fühlkopf (18) aufweist.

12. Mikrowellengerät nach Anspruch 1, **dadurch gekennzeichnet,** daß der Fühlkopf (18) annähernd in der Mitte der Auflagefläche (9) vorgesehen ist.

13. Mikrowellengerät nach Anspruch 11, **dadurch gekennzeichnet,** daß der Fühlkopf (18) aus stark wärmeleitendem Material eine Schutzbeschichtung (19), vorzugsweise aus einem Edel- oder Buntmetall, aufweist.

14. Mikrowellengerät nach einem der Ansprüche 1 bis 13, **dadurch gekennzeichnet,** daß der Temperaturfühler (17) mit einer Schaltsteuereinrichtung (24,38) für den Mikrowellenerzeuger (29) in Verbindung steht, und daß ein Glied (39) vorgesehen ist, das in Abhängigkeit von der Geschwindigkeit des Temperaturanstiegs im Beutel (2) bei Erreichen eines vorbestimmten Geschwindigkeitsgrenzwertes den Mikrowellenerzeuger (29) über eine, gegebenenfalls vorbestimmte, Zeitdauer ab- und nach Verstreichen dieser Zeitdauer wieder einschaltet.

15. Mikrowellengerät nach Anspruch 1, wobei an der Aufnahmevorrichtung (7) Mikrowellen-Abschirmflächen (A) zumindest für den Anschlußbereich (47) des Beutels (2) vorgesehen sind, **dadurch gekennzeichnet,** daß die Abschirmfläche für die Beuteloberseite (6) eine an der Schale (8, 8') zwischen einer Beuteleinlegestellung (e) und einer Abschirmstellung (a) beweglich angeordnete Platte (40) ist, die in der Abschirmstellung (a) die Oberseite (6) des Beutels (2) teilweise zumindest im Anschlußbereich (47) des Beutels (2) überdeckt.

16. Mikrowellengerät nach Anspruch 15, **dadurch gekennzeichnet,** daß die Platte (40) um ein Scharnier klappbar angeordnet ist, dessen Scharnierachse (42) zwischen gegenüberliegenden Stützrändern (10) verläuft.

17. Mikrowellengerät nach Anspruch 15, **dadurch gekennzeichnet,** daß die Schale (8) Anschläge (45, 46) für die Platte (40) aufweist, an denen diese in beiden Stellungen (a, e) abgestützt ist.

18. Mikrowellengerät nach den Ansprüchen 15 bis 17, **dadurch gekennzeichnet,** daß die Scharnierachse (42) die Platte (40) in zwei verschieden große Plattenteile (40a, 40b) unterteilt, und daß der Massenschwerpunkt der Platte (40) bzw. des größeren Plattenteils (40a) in der Beuteleinlegestellung (e) - in einer vertikalen Projektion - an der Seite der Scharnierachse (42) liegt, zu der sich der größere Plattenteil (40a) - bei der Bewegung der Platte (40) in die Abschirmstellung (a) -bewegt.

19. Mikrowellengerät nach Anspruch 16, **dadurch gekennzeichnet,** daß das Scharnier aus Zapfen (44) an den Seiten der Platte (40) und aus Vertiefungen (43) in den Stützrändern (10) besteht.

20. Mikrowellengerät nach Anspruch 15, **dadurch gekennzeichnet,** daß an der Unterseite der Schale (8) eine in der Größe annähernd der Platte (40) entsprechende Abschirmplatte (41) angebracht ist, die in etwa mit der in der Abschirmstellung (a) befindlichen Platte (40) deckungsgleich liegt.

21. Mikrowellengerät nach einem der Ansprüche 1 bis 15, **dadurch gekennzeichnet,** daß die freie Länge (L, L1) der Auflagefläche (9) zur Anpassung an unterschiedliche Beutelgrößen verstellbar ist.

22. Mikrowellengerät nach Anspruch 21, **dadurch gekennzeichnet,** daß die Schale (8) eine Auflagefläche (9) mit einer auf die Länge der größten Beutelgröße abgestimmten freien Länge (L) besitzt, und daß an der Schale (8) oberhalb der Auflagefläche (9) ein Querteilelement (48) lösbar und in Längsrichtung verstellbar angeordnet ist.

23. Mikrowellengerät nach Anspruch 22, **dadurch gekennzeichnet,** daß das Querteilelement (48) ein an gegenüberliegenden Stützrändern (10) festgelegter und den Stützrändern (10) entlang verschiebbarer, sich oberhalb der Auflagefläche (9) quer erstreckender Klemmbügel aus Kunststoff ist.

24. Mikrowellengerät nach Anspruch 23, **dadurch gekennzeichnet,** daß der Klemmbügel in etwa W-förmige Gestalt hat und an den Stützrändern (10) mit Klammerenden (49) festgeklemmt ist.

25. Mikrowellengerät nach Anspruch 24, **dadurch gekennzeichnet,** daß bei zumindest einem Klammerende (49) eine nach außen stehende Abdrücklasche (51) vorgesehen ist.

26. Mikrowellengerät nach einem der Ansprüche 1 bis 25, **dadurch gekennzeichnet,** daß an den Stützrändern (10) der Schale (8) in etwa parallel zur Auflagefläche (9) nach außen ragende Randflansche (53) angeformt sind.

FIG.1

FIG. 2

FIG. 3

FIG. 4

FIG. 5